# EUROPEAN PATENT APPLICATION

(11) **EP 3 305 193 A1**
(43) Date of publication of application: **11.04.2018**
(21) Application number: 16803018.7
(22) Date of filing: 13.05.2016
(51) Int. Cl.: A61B 5/107, G06M 3/00

(54) **POSTURE DETECTION DEVICE, SPECTACLE-TYPE ELECTRONIC DEVICE, POSTURE DETECTION METHOD, AND PROGRAM**

(30) Priority: 29.05.2015 JP 2015110241
(71) Applicant: Alps Electric Co., Ltd., Tokyo 145-8501 (JP)
(72) Inventor: YAMADA, Yukimitsu, Tokyo 145-8501 (JP)
(74) Representative: TBK
(86) International application number: PCT/JP2016/064268
(87) International publication number: WO 2016/194581

(57) **Abstract**

Provided is a posture detection apparatus that can detect the posture of a target with lower power consumption, on a smaller scale, and with lower cost. A step count detection unit 951 detects one step (a step count) on the basis of the acceleration from an acceleration sensor 71, and outputs the detection result to an acceleration accumulation unit 953. The acceleration accumulation unit 953 calculates, within a period of time corresponding to the one step detected by the step count detection unit 951, an acceleration cumulative value (simple posture pitch angle) by accumulating the acceleration detected by the acceleration sensor 71. The posture detection unit 955 detects, on the basis of the acceleration cumulative value calculated by the acceleration accumulation unit 953, the posture of a portion for which the acceleration sensor 71 is provided.

## Description

### Technical Field

The present invention relates to a posture detection apparatus, a glasses-type electronic device, a posture detection method, and a program for detecting the posture of a target such as a portion of a human body.

### Background Art

For example, the posture of a human body at the time of walking may be a parameter indicating a mental activity state or a physical condition of the human body.

Such a posture at the time of walking can be determined by, for example, detecting a displacement with respect to the axis of a body at the time of walking.

By the way, the acceleration output from an acceleration sensor is affected by acceleration occurring at the time of walking other than gravity, and thus the posture cannot be determined only using the acceleration, and angular velocity that is not affected by the acceleration is necessary.

At present, as a method for calculating an attitude angle of a rigid body in action, a method for calculating an attitude angle by integrating the angular velocity measured by a gyro sensor is widely used.

### Summary of Invention

### Technical Problem

However, there is a problem in that a gyro sensor has higher power consumption, a larger scale, and higher cost than an acceleration sensor.

In contrast, when the acceleration detected by an acceleration sensor at the time of walking is simply used as is, it is affected by acceleration caused by walking, and thus there is a problem in that a large posture detection error occurs.

The present invention has been made in light of such circumstances, and the object thereof is to provide a posture detection apparatus, a glasses-type electronic device, a posture detection method, and a program that enable detection of the posture of a target using a configuration having lower power consumption, a smaller scale, and lower cost.

### Solution to Problem

In order to solve the problems of the above-described existing technology and to achieve the above-described object, a posture detection apparatus according to the present invention has accumulation means that accumulates, within a predetermined period, a plurality of accelerations corresponding to movements of a portion that is a detection target, the accelerations being detected at predetermined time intervals, and posture detection means that detects a posture of the portion on the basis of the accumulated acceleration.

With this configuration, information closely analogous to information detected using a gyro sensor can be obtained by accumulating, at the predetermined time intervals, the plurality of accelerations corresponding to the movements of the portion that is the detection target at the accumulation means. Thus, the posture of the portion can be detected on the basis of the accumulated acceleration at the posture detection means. Posture detection can be performed on the basis of only acceleration in this manner, and thus the posture of the target can be detected using a configuration having lower power consumption, a smaller scale, and lower cost.

Preferably, the accumulation means of the posture detection apparatus according to the present invention accumulates the accelerations in a plurality of directions on a direction basis, and the posture detection means detects the posture of the predetermined portion on the basis of the accelerations accumulated regarding the plurality of directions.

With this configuration, the posture of the target can be detected in the plurality of directions.

Preferably, the posture detection means of the posture detection apparatus according to the present invention detects an inclination of the portion with respect to a predetermined axis.

With this configuration, the posture of the portion of the target can be detected as the inclination of the portion with respect to the predetermined axis.

Preferably, the posture detection apparatus according to the present invention has step count detection means that detects one step of the human body on the basis of the accelerations, the accumulation means accumulates the accelerations using a period of time corresponding to the one step as the predetermined period, and the posture detection means detects an inclination with respect to the axis of the human body on the basis of a cumulative value of the accelerations within the period of time corresponding to the one step.

With this configuration, the inclination with respect to the axis of the human body at the time when the human body is walking can be detected.

Preferably, the posture detection apparatus according to the present invention further has acceleration detection means that detects the accelerations. With this configuration m, the accelerations can be obtained by the acceleration detection means.

Preferably, the acceleration detection means of the posture detection apparatus according to the present invention is provided on or near the head of a human body.

With this configuration, the posture of the human body during exercise can be detected with high accuracy by providing the acceleration detection means on or near the head.

A glasses-type electronic device according to the present invention is provided with the above-described posture detection apparatus.

A posture detection method according to the present invention has an accumulation step for accumulating, within a predetermined period, a plurality of accelerations corresponding to movements of a portion that is a detection target, the accelerations being detected at predetermined time intervals, and a posture detection step for detecting a posture of the portion on the basis of the accumulated acceleration.

A program according to the present invention causes a computer to execute: an accumulation process for accumulating, within a predetermined period, a plurality of accelerations corresponding to movements of a portion that is a detection target, the accelerations being detected at predetermined time intervals, and a posture detection process for detecting a posture of the portion on the basis of the accumulated acceleration.

### Advantageous Effects of Invention

According to the present invention, a posture detection apparatus, a glasses-type electronic device, a posture detection method, and a program that enable detection of the posture of a target using a configuration having lower power consumption, a smaller scale, and lower cost can be provided. Brief Description of Drawings
[Fig. 1] Fig. 1 is an external perspective view of glasses according to an embodiment of the present invention.
[Fig. 2] Fig. 2 is a functional block diagram according to posture detection by the glasses illustrated in Fig. 1.
[Fig. 3] Fig. 3 is a functional block diagram of a processing unit of the glasses illustrated in Fig. 2.
[Fig. 4] Fig. 4 is a diagram illustrating a simple posture pitch angle generated by the glasses illustrated in Fig. 1 and an ideal posture pitch angle generated using a method using a conventional gyro.
[Fig. 5] Fig. 5 is a diagram illustrating the relationship between the simple posture pitch angle and the ideal posture pitch angle illustrated in Fig. 4.
[Fig. 6] Fig. 6 is a flowchart of acceleration detection by the processing unit illustrated in Fig. 2.
[Fig. 7] Fig. 7 is a free chart of an acceleration cumulative value (simple posture pitch angle) generation process by the processing unit of the glasses illustrated in Fig. 2.

### Description of Embodiments

The inventor found that the posture of a portion of a target can be detected, without the use of angular acceleration, by accumulating accelerations of the portion at predetermined time intervals.

In the present embodiment, a case will be exemplified in which the acceleration from an acceleration sensor provided for the head of a human body is accumulated over a period of time corresponding to one step, and the displacement of the head from the axis of the body is determined on the basis of the cumulative value, and the posture of the head is determined from the displacement.

In the following, glasses 1 according to the embodiment of the present invention will be described.

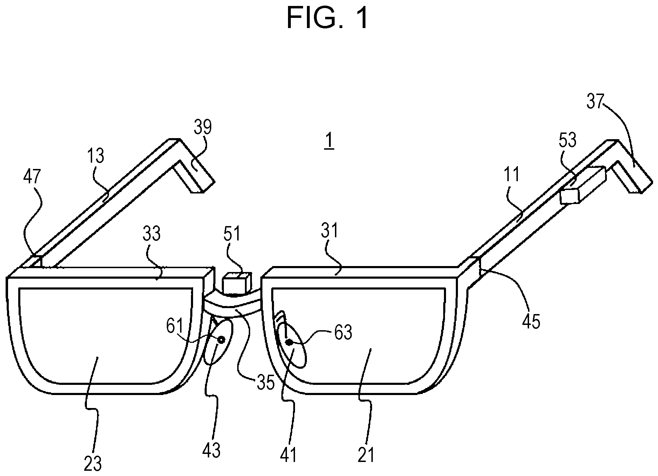

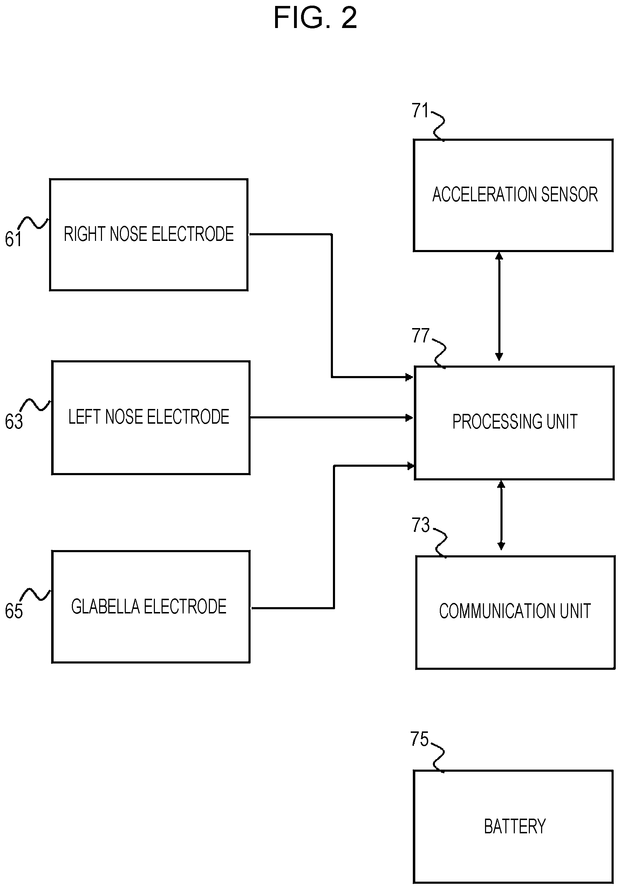

Fig. 1 is an external perspective view of the glasses 1 according to the embodiment of the present invention. Fig. 2 is a functional block diagram of the glasses 1 illustrated in Fig. 1.

As illustrated in Fig. 1, the glasses 1 have, for example, temples 11 and 13 that can be worn over a user's ears, rims 31 and 33 by which lenses 21 and 23 are fixed, a bridge 35 that is interposed between the rims 31 and 33, and nose pads 41 and 43. The tips of the rims 31 and 33 are called temple tips 37 and 39. In addition, hinges 45 and 47 are provided between the temples 11 and 13 and the rims 31 and 33.

The temples 11 and 13, the rims 31 and 33, the bridge 35 interposed between the rims 31 and 33, the nose pads 41 and 43, the temple tips 37 and 39, and the hinges 45 and 47 are an example of a glasses-type frame according to the present invention.

As illustrated in Fig. 2, a storage box 51 is provided between the nose pads 41 and 43.

In addition, a storage box 53 is fixed on the side where the temple tip 37 of the temple 11 is provided.

A right nose electrode 61 is provided on a surface of the nose pad 41, and a left nose electrode 63 is provided on a surface of the nose pad 43.

In a state in which the user is wearing the glasses 1, the right nose electrode 61 is in contact with (pressed against) a right side surface with respect to the bridge of the nose of the user, and detects an eye electric potential that is the electric potential of the skin that is in contact.

In the state in which the user is wearing the glasses 1, the left nose electrode 63 is in contact with a left side surface with respect to the bridge of the nose of the user, and detects an eye electric potential that is the electric potential of the skin that is in contact.

The right nose electrode 61 and the left nose electrode 63 are symmetrically placed right and left when the nose of the user is viewed from the front while the user is wearing the glasses 1.

At the storage box 51, there is provided a glabella electrode 65 that is in contact with the root of the nose or the glabella of the user and detects the electric potential of the skin that is in contact in the state in which the user is wearing the glasses 1.

The right nose electrode 61, the left nose electrode 63, and the glabella electrode 65 are, for example, made of stainless steel or titanium.

The right nose electrode 61, the left nose electrode 63, and the glabella electrode 65 are formed in shapes that are appropriate for the shapes of human body portions to be in contact.

The storage box 53 has a storage space inside thereof, and an acceleration sensor 71, a communication unit 73, a battery 75, and a processing unit 77 are stored in the storage space.

The storage box 51 and the storage box 53 are electrically connected by wiring such as a printed board.

The acceleration sensor 71 is an acceleration sensor for three axes: X, Y, and Z, and outputs, for each axis, detected acceleration to the processing unit 77. The acceleration sensor 71 detects acceleration at predetermined detection time intervals. The acceleration sensor 71 stores the detected acceleration in a memory (not illustrated).

In the present embodiment, when the user is wearing the glasses 1, the acceleration sensor 71 is positioned near his or her ear of his or her head such that the movement of his or her head is appropriately detected.

The communication unit 73 performs wireless communication using for example Bluetooth® or a wireless LAN, and can transmit, to an external apparatus, the eye electric potentials input from the right nose electrode 61, the left nose electrode 63, and the glabella electrode 65, the acceleration input from the acceleration sensor 71, and so on. High functional processing using high processing performance and a memory capacity can be realized.

The processing unit 77 generates information regarding the user on the basis of the eye electric potentials input from the right nose electrode 61, the left nose electrode 63, and the glabella electrode 65, and the acceleration input from the acceleration sensor 71.

The eye electric potentials (skin potentials) input from the right nose electrode 61, the left nose electrode 63, and the glabella electrode 65, and the acceleration input from the acceleration sensor 71 are electric potentials corresponding to perspiration and movement of the user, and reflect the user's physical condition or mental state. Thus, by preparing, in advance, reference data in which eye electric potentials and acceleration are associated with the user's physical conditions or mental states, the processing circuit 77 can detect the user's physical condition or mental state by comparing the input electric potentials and acceleration of the user with the above-described reference data.

The cornea side of an eyeball is positively charged, and the retina side is negatively charged. Thus, when the user's line of sight moves upward, the eye electric potential of the right nose electrode 61 with reference to the eye electric potential of the glabella electrode 65, and the eye electric potential of the left nose electrode 63 with reference to the glabella electrode 65 become negative.

In contrast, when the user's line of sight moves downward, the eye electric potential of the right nose electrode 61 with reference to the eye electric potential of the glabella electrode 65, and the eye electric potential of the left nose electrode 63 with reference to the eye electric potential of the glabella electrode 65 become positive.

When the user's line of sight moves rightward, the eye electric potential of the right nose electrode 61 with reference to the glabella electrode 65 becomes negative, and the eye electric potential of the left nose electrode 63 with reference to the glabella electrode 65 becomes positive.

When the user's line of sight moves leftward, the eye electric potential of the right nose electrode 61 with reference to the glabella electrode 65 becomes positive, and the eye electric potential of the left nose electrode 63 with reference to the glabella electrode 65 becomes negative.

Note that instead of detection of the eye electric potential of the right nose electrode 61 with reference to the eye electric potential of the glabella electrode 65 the glabella electrode 65, the eye electric potential of the glabella electrode 65 with reference to a reference electrode may be subtracted from the eye electric potential of the right nose electrode 61 with reference to the reference electrode. Likewise, instead of detection of the eye electric potential of the left nose electrode 63 with reference to the eye electric potential of the glabella electrode 65, the eye electric potential of the glabella electrode 65 with reference to the reference electrode may be subtracted from the eye electric potential of the left nose electrode 63 with reference to the reference electrode. A ground electrode may be used as the reference electrode.

In this manner, in the case where a positive detection eye electric potential is indicated, it can be detected that the user's line of sight is directed upward. In the case where a negative detection eye electric potential is indicated, it can be detected that the user's line of sight is directed downward.

Furthermore, in the case where the eye electric potential from the right nose electrode 61 is negative and the eye electric potential from the left nose electrode 63 is positive, it can be detected that the user's line of sight is directed rightward, and in the case where the eye electric potential from the right nose electrode 61 is positive and the eye electric potential from the left nose electrode 63 is negative, it can be detected that the user's line of sight is directed leftward.

In the following, a function regarding human-body posture detection by the glasses 1 will be described.

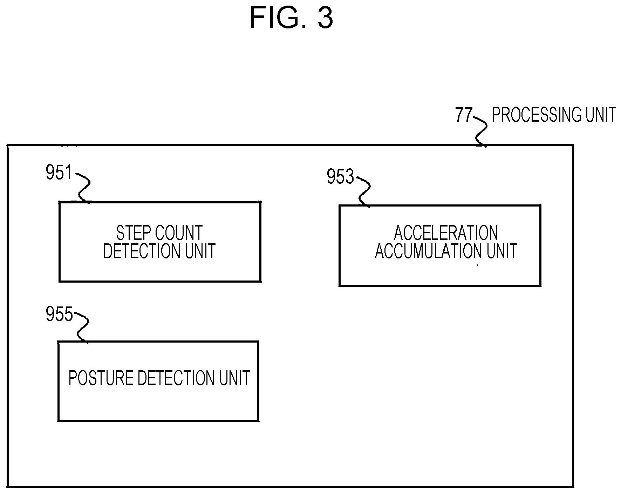

Fig. 3 is a functional block diagram regarding posture detection by the processing unit 77 illustrated in Fig. 2.

As illustrated in Fig. 3, the processing unit 77 includes, for example, a step count detection unit 951, an acceleration accumulation unit 953, and a posture detection unit 955.

The functions of the units of the processing unit 77 may be realized by executing programs at a processing circuit, or at least some of the functions may be realized using hardware.

The step count detection unit 951 detects one step (a step count) on the basis of the acceleration from the acceleration sensor 71, and outputs the detection result to the acceleration accumulation unit 953.

The step count detection unit 951 detects one step, for example, on the condition that a combined value of three-axis accelerations detected by the acceleration sensor 71 becomes lower than 1G and thereafter becomes higher than 1G.

The acceleration accumulation unit 953 calculates, within a period of time corresponding to the one step detected by the step count detection unit 951, acceleration cumulative values by accumulating the accelerations detected by the acceleration sensor 71.

In this manner, the acceleration accumulation unit 953 integrates, for each axis, acceleration in units of one step, and calculates an acceleration cumulative value (simple posture pitch angle) that is the integral of the acceleration. The direction in which his or her head is inclined with respect to the axis of his or her body can be determined on the basis of the acceleration cumulative values, the acceleration sensor 71 being provided for his or her head.

Note that an up-down action occurs when he or she walks. The acceleration accumulation unit 953 can calculate a roll in addition to a pitch on the basis of the acceleration cumulative value for a predetermined axis.

The acceleration accumulation unit 953 can determine, on the basis of the direction of acceleration, how the acceleration sensor 71 (his or her head) is inclined.

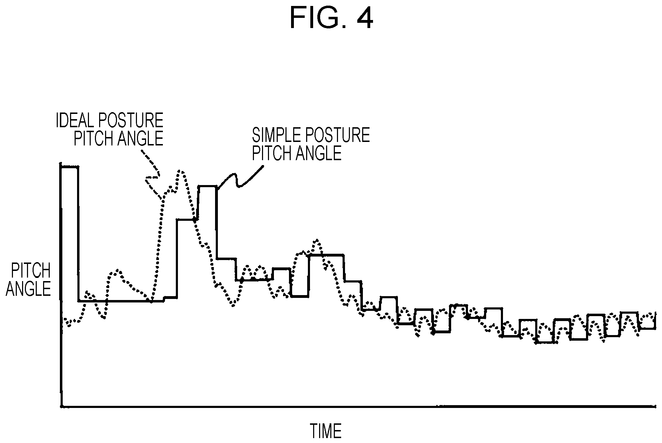

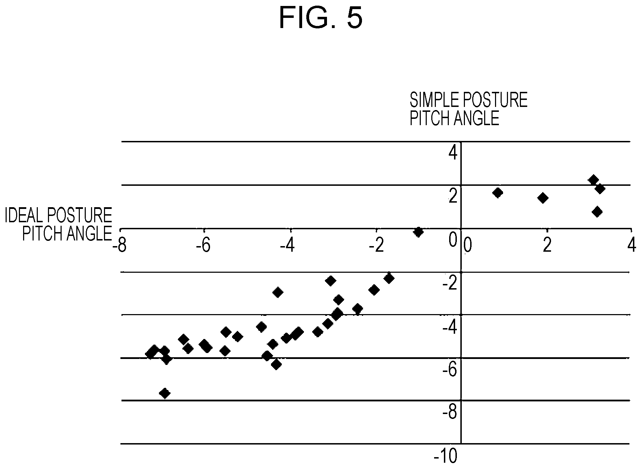

Fig. 4 is a diagram illustrating a one-axis simple posture pitch angle generated by the glasses 1 illustrated in Fig. 1 and an ideal posture pitch angle generated using a method using a conventional gyro. Fig. 5 is a diagram illustrating the relationship between the one-axis simple posture pitch angle and the ideal posture pitch angle illustrated in Fig. 4.

As illustrated in Figs. 4 and 5, the acceleration cumulative value (simple posture pitch angle) generated by the acceleration accumulation unit 953 is closely analogous to the ideal posture pitch angle obtained by using the gyro. Thus, in order to detect the posture of the human body, the simple posture pitch angle can be used instead of the ideal posture pitch angle.

The posture detection unit 955 detects, on the basis of the acceleration cumulative values calculated by the acceleration accumulation unit 953, the posture of a portion for which the acceleration sensor 71 is provided. The posture is an inclination from the axis of his or her body, that is, a pitch angle.

From the detected posture of the portion (his or her head) as described above, the posture detection unit 955 detects the inclination of his or her body for every step and determines the left-and-right balance of his or her walking posture.

Through a posture determination at the time of walking, his or her age can be determined on the basis of, for example, the displacement and inclination of his or her body. For example, an elderly person has a small displacement.

That is, since a human walks while losing balance between left and right, a health state of the human can also be checked on the basis of the posture of his or her head by looking at the movement of his or her head. When the displacement from the axis of his or her body is large at the time of walking, the correlation between the displacement and a disease is determined.

The case where the acceleration sensor 71 is provided for his or her head has been exemplified in the present embodiment; however, the acceleration sensor 71 may also be provided for another portion such as a foot of a human body.

The posture detection unit 955 uses the cumulative values of acceleration within one step and generated by the acceleration accumulation unit 953 in the above-described example, but may also use the mean value of the acceleration.

In the following, an operation of the glasses 1 according to the embodiment of the present invention will be described.

### [Acceleration Detection Processing]

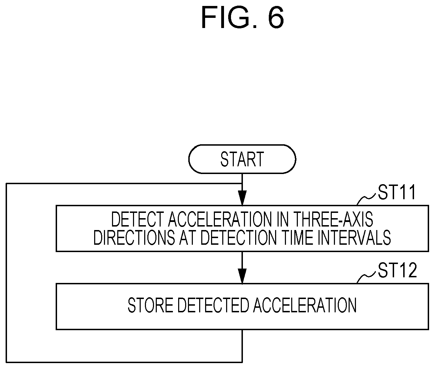

Fig. 6 is a flowchart of acceleration detection by the processing unit 77 illustrated in Fig. 2.

### Step ST11:

The acceleration sensor 71 detects acceleration in the three-axis directions at predetermined time intervals, the three axes being X, Y, and Z.

### Step ST12:

The acceleration sensor 71 stores, in the memory, the acceleration detected in the three-axis directions in step ST11.

### [Simple Posture Pitch Angle Generation]

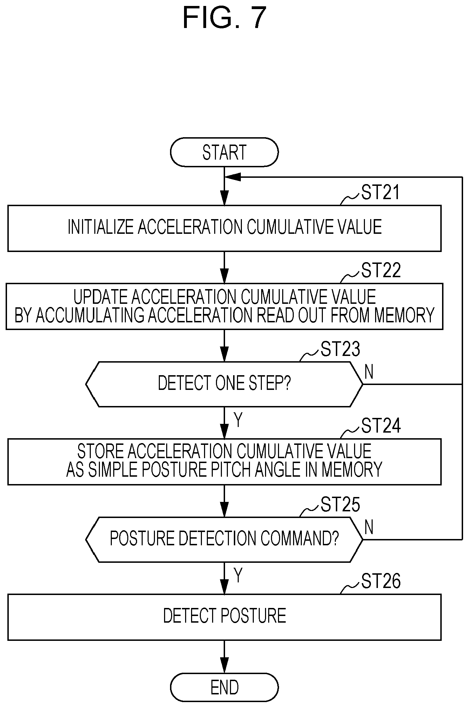

Fig. 7 is a free chart of an acceleration cumulative value (simple posture pitch angle) generation process by the processing unit of the glasses illustrated in Fig. 2.

The process illustrated in Fig. 7 is performed, for example, after the process illustrated in Fig. 6 is completed.

### Step ST21:

The acceleration accumulation unit 953 initializes an acceleration cumulative value (for example, 0).

### Step ST22:

The acceleration accumulation unit 953 reads out, one after another, acceleration written into the memory in accordance with the flowchart illustrated in Fig. 6, and adds (accumulates) this to the acceleration cumulative value (simple posture pitch angle).

### Step ST23:

It is determined whether the step count detection unit 951 has detected one step (a step count) on the basis of the acceleration from the acceleration sensor 71. In the case where it is determined that one step has been detected, the process proceeds to step ST24. The step count detection unit 951 detects one step, for example, on the condition that a combined value of three-axis accelerations detected by the acceleration sensor 71 becomes lower than 1G and thereafter becomes higher than 1G.

### Step ST24:

The acceleration accumulation unit 953 stores, in the memory, the latest acceleration cumulative value as a simple posture pitch angle.

### Step ST25:

The posture detection unit 955 determines whether a posture detection command has been received. In the case where it is determined that a posture detection command has been received, the process proceeds to step ST26. Otherwise the process returns to step ST21.

### Step ST26:

The posture detection unit 955 reads out the simple posture pitch angle from the memory, and detects, on the basis of this, the posture of a portion for which the acceleration sensor 71 is provided. The posture is an inclination from the axis of his or her body.

From the posture of the detected portion (his or her head) as described above, the posture detection unit 955 detects the inclination of his or her body for every step and determines the left-and-right balance of his or her walking posture.

Through a posture determination at the time of walking, various types of information such as the user's physical condition can be determined on the basis of, for example, the displacement and inclination of his or her body.

Note that the above-described process of Fig. 7 may be individually performed on the accelerations for each of the three axes from the acceleration sensor 71, or may also be performed on combined acceleration obtained by combining these accelerations.

As described above, the glasses 1 generate the user's simple posture pitch angle at the time of walking using the algorithms illustrated in Figs. 6 and 7, and thus no gyro sensor has to be used, and the user's posture can be detected with lower power consumption and lower cost and on a smaller scale.

In addition, as illustrated in Figs. 4 and 5, the glasses 1 can achieve a necessary detection accuracy without significant degradation of the detection accuracy of the user's posture, compared with the case where a gyro sensor is used.

In addition, since the acceleration sensor 71, the communication unit 73, the battery 75, and the processing unit 77 are stored in the storage box 53, the glasses 1 are superior in design and can be worn daily without awkwardness.

In addition, high functional processing using the high processing performance and memory capacity of an external apparatus such as a portable communication apparatus can be realized using the glasses 1 by transmitting signals (data) to the external apparatus via the communication unit 73 within the storage box 53.

The present invention is not limited to the above-described embodiment.

That is, those skilled in the art may add various changes to, make combinations and subcombinations from among, and perform replacements for the constituent elements of the above-described embodiment within the technical scope of the present invention or its equivalent scope.

In the above-described embodiment, the case where the posture of a human body is detected from his or her walking action has been exemplified; however, his or her posture or the like may be detected on the basis of an action other than the walking action, or generation of a portion of a certain target in action other than a human body may be detected.

In addition, the case where the acceleration sensor 71 is provided in the storage box 53 positioned on the side where the temple tip 37 of the temple 11 is provided has been exemplified in the above-described embodiment; however, the acceleration sensor 71 may also be provided at another position of the glasses 1. In addition, a plurality of acceleration sensors may be provided at different positions of the glasses 1.

In addition, the case where the present invention is applied to the glasses 1 provided with the lenses 21 and 23 has been exemplified in the above-described embodiment; however, the present invention may also be applied to eyewear or the like having no lens.

### Industrial Applicability

The present invention can be used for a posture detection apparatus that detects the posture of a portion of a human body.

### Reference Signs List

- 1: glasses
- 11, 13: temple
- 21, 23: lens
- 31, 33: rim
- 37, 39: temple tip
- 35: bridge
- 45, 47: hinge
- 51, 53: storage box
- 61: right nose electrode
- 63: left nose electrode
- 65: glabella electrode
- 71: acceleration sensor
- 73: communication unit
- 75: battery
- 77: processing unit
- 951: step count detection unit
- 953: acceleration accumulation unit
- 955: posture detection unit

## Claims

1. A posture detection apparatus comprising:
accumulation means that accumulates, within a predetermined period, a plurality of accelerations corresponding to movements of a portion that is a detection target, the accelerations being detected at predetermined time intervals; and
posture detection means that detects a posture of the portion on the basis of the accumulated acceleration.

2. The posture detection apparatus according to Claim 1, wherein
the accumulation means accumulates the accelerations in a plurality of directions on a direction basis, and
the posture detection means detects the posture of the predetermined portion on the basis of the accelerations accumulated regarding the plurality of directions.

3. The posture detection apparatus according to Claim 1 or 2, wherein
the posture detection means detects an inclination of the portion with respect to a predetermined axis.

4. The posture detection apparatus according to any one of Claims 1 to 3, comprising:
step count detection means that detects one step of the human body on the basis of the accelerations, wherein
the accumulation means accumulates the accelerations using a period of time corresponding to the one step as the predetermined period, and
the posture detection means detects an inclination with respect to the axis of the human body on the basis of a cumulative value of the accelerations within the period of time corresponding to the one step.

5. The posture detection apparatus according to any one of Claims 1 to 4, further comprising:
acceleration detection means that detects the accelerations.

6. The posture detection apparatus according to any one of Claims 1 to 5, wherein
the acceleration detection means is provided on or near the head of a human body.

7. A glasses-type electronic device comprising: the posture detection apparatus according to any one of Claims 1 to 6.

8. A posture detection method comprising:
an accumulation step for accumulating, within a predetermined period, a plurality of accelerations corresponding to movements of a portion that is a detection target, the accelerations being detected at predetermined time intervals; and
a posture detection step for detecting a posture of the portion on the basis of the accumulated acceleration.

9. A program causing a computer to execute:
an accumulation process for accumulating, within a predetermined period, a plurality of accelerations corresponding to movements of a portion that is a detection target, the accelerations being detected at predetermined time intervals; and
a posture detection process for detecting a posture of the portion on the basis of the accumulated acceleration.
